# EUROPÄISCHE PATENTSCHRIFT

(11) **EP 0 750 196 B1**
(45) Veröffentlichungstag und Bekanntmachung des Hinweises auf die Patenterteilung: **12.09.2001**
(21) Anmeldenummer: 96109903.3
(22) Anmeldetag: 20.06.1996
(51) Int. Cl.: G01N 33/52

(54) **Mehrschichtiges Analysenelement zur Bestimmung eines Analyten in einer Flüssigkeit**
Multi-layer analytical element for the determination of an analyte in a fluid
Elément d'analyse à plusieurs couches pour la détermination d'un analyte dans un fluide

(30) Priorität: 24.06.1995 DE 19523049
(43) Veröffentlichungstag der Anmeldung: 27.12.1996
(73) Patentinhaber: Roche Diagnostics GmbH, 68298 Mannheim (DE)
(72) Erfinder: Zimmer, Volker, 67069 Ludwigshafen (DE); Macho, Heinz, 64658 Fürth (DE); Lerch, Rolf, 68549 Ilvesheim (DE)

(56) Entgegenhaltungen:
- EP-A- 0 045 476
- EP-A- 0 271 854
- EP-A- 0 327 918
- US-A- 4 337 222
- US-A- 4 604 264

## Beschreibung

Die Erfindung betrifft ein mehrschichtiges Analysenelement zur Bestimmung eines Analyten in einer Flüssigkeit enthaltend nebeneinander angeordnet eine Probenauftragszone und eine Detektionszone, wobei die Detektionszone ein Reagenz enthält, das mit dem zu bestimmenden Analyt oder einer hiervon abgeleiteten Substanz ein detektierbares Signal bildet. Die Erfindung betrifft außerdem einen stapelartigen Materialverbund aus einem Vlies und einer porösen Membran sowie die Verwendung eines derartigen stapelartigen Materialverbundes zur Herstellung eines mehrschichtigen Analyseelementes. Außerdem ist Gegenstand der Erfindung ein Verfahren zur Bestimmung eines Analyts in einer Flüssigkeit mittels eines mehrschichtigen Analyseelementes.

Eine mehrschichtige Analysenvorrichtung ist beispielsweise aus US-A-4,292,272 bekannt, bei der Probenaufgabe- und Detektionszone übereinander in einer Achse angeordnet sind. Eine hydrophilisierte Spreitschicht aus Gewebe ist darin auf eine Reagenz enthaltende Schicht laminiert. Diese Reagenz enthaltende Schicht ist ein Film aus Gelatine, Polyvinylalkohol, Polyvinylpyrolidon, Agarose, Natriumpolyvinylbenzolsulfonat oder ähnlichem. Zur Herstellung solcher Filmschichten bedarf es erheblichen know hows, so daß es nicht verwunderlich ist, daß diese Technologie vor allem von Firmen aus der fotografischen Filmbranche entwickelt wurde und heute noch angewandt wird. Die Herstellung solcher Analysenvorrichtungen beinhaltet ein hohes Montagerisiko, da alle Reagenzien zusammen mit der Filmlösung oder Suspension verarbeitet werden. Für die Untersuchung gefärbter Flüssigkeiten müssen oft zusätzliche optische Sperrschichten verwendet werden, um zu verhindern, daß die Flüssigkeitsfarbe auf der Detektionsseite, die der Probenaufgabeseite gegenüber liegt, stört.

In WO-A-9217768 werden mehrschichtige Analysenelemente beschrieben, bei denen zwei oder mehr Schichten porösen Papiers oder Polymermaterials durch eine dazwischenliegende Schicht zusammengehalten werden. Mindestens eine der Schichten enthält Reagenz, das in die Schicht inkorporiert worden ist, bevor die Schichten zusammengefügt wurden. Es werden nur Testvorrichtungen mit Papier als Schichtenmaterialien näher beschrieben. Die konkrete Natur möglicher Polymermaterialien wird nicht angegeben. Insofern treten in den vorgeschlagenen Testvorrichtungen beim Flüssigkeitsübertritt von einer in eine andere Schicht sowohl horizontaler als auch vertikaler Flüssigkeitstransport ohne wesentliche räumliche und zeitliche Trennung nebeneinander in bedeutendem Ausmaß auf. Dies hat zur Folge, daß Reagenz in der dieses enthaltenden Schicht von der Probenflüssigkeitsfront gelöst und mittransportiert wird. In der Flüssigkeitsfront befindet sich in der Regel mehr Substanz gelöst, als in der nachfolgenden Flüssigkeit. Dies wird auch als Chromatographieeffekt bezeichnet. Bei sehr schnellen Umsätzen reagiert der nachzuweisende Analyt bereits in der Flüssigkeitsfront. Über die Wegstrecke der mit Flüssigkeit zu füllenden Schicht wird die Probe dann an Analyt abgereichert. Beide Effekte führen zu inhomogener Signalbildung.

EP-A-0 271 854 betrifft ein mehrschichtiges Analysenelement für die analytische Bestimmung von Bestandteilen von Körperflüssigkeiten, bei dem sich auf einer Tragschicht nebeneinander eine Aufgabe-, eine Nachweis- und eine Saugzone befinden. Die Aufgabezone ist mit der Saugzone durch eine kapillaraktive Transportstrecke verbunden. Als Material für die Transportstrecke sind als beispielhafte Möglichkeiten ein Vlies oder Gewebe genannt. Polyamid wird als besonders geeignet bezeichnet. Die Transportstrecke langt vom Anfang der Aufgabezone über die Nachweiszone hinaus. In der Nachweiszone befinden sich eine oder mehrere Reaktionsschichten so angeordnet, daß sie in Flüssigkeitskontakt mit einer in der Transportstrecke transportierten Flüssigkeit stehen. Die Reaktionsschicht kann direkt auf der die Transportstrecke bildenden Schicht aufliegen. Sie kann aus Papier, Vlies, Gel, Gelatine oder einem porösen Kunststoffmaterial bestehen. Gemäß der europäischen Anmeldung sollen Saugkraft, Saugvolumen und die Sauggeschwindigkeiten der Schichten so aufeinander abgestimmt sein, daß Flüssigkeit in der Transportschicht der Nachweiszone so lange zur Verfügung steht, bis die Reaktionsschichten eine definierte Flüssigkeitsmenge aufgenommen haben, danach aber der Flüssigkeitskontakt zwischen Reaktions- und Transportschicht durch die Wirkung der Saugzone unterbrochen wird. Hierbei wird die Flüssigkeit aus der Transportstrecke herausgesaugt. Wie aus dem Beispiel ersichtlich, werden die Reaktionsschichten vor der Montage mit Reagenz behandelt. Hierdurch ergibt sich auch hier ein Montagerisiko, weil dann, wenn die mit teuerem Reagenz behandelten Schichten fehlerhaft aufmontiert werden, mit hohem Kostenaufwand hergestellte Zwischenprodukte verloren sind. Die Abstimmung der Eigenschaften der Materialien aller Funktionszonen ist durch die hohe Anzahl der Funktionszonen recht kompliziert. Weiterhin ergeben sich durch den ständigen Flüssigkeitsstrom in der Transportstrecke zum Füllen der Reaktionsschicht und zum anschließenden Abbruch des Flüssigkeitskontaktes zwischen Transportschicht und Reaktionsschicht strömungsbedingte Störungen in der Signalbildung der Reaktionsschicht. Eine flächenhomogene Farbreaktion kann so nicht erwartet werden.

Der Erfindung lag deshalb die Aufgabe zugrunde, kompakte, einfach, universal und billig herzustellende Analysenelemente zur Verfügung zu stellen, bei denen eine flächenhomogene Reaktion in der Reaktionsschicht ohne chemische Fixierung des dort vorliegenden Reagenzes erfolgt.

Diese Aufgabe wird durch den Gegenstand der Erfindung, wie er in den Patentansprüchen näher charakterisiert ist, gelöst.

Gegenstand der Erfindung ist ein mehrschichtiges Analysenelement zur Bestimmung eines Analyten in einer Flüssigkeit enthaltend nebeneinander angeordnet eine Probenauftragszone und eine Detektionszone, wobei die Detektionszone ein Reagenz enthält, das mit dem zu bestimmenden Analyt oder einer hiervon abgeleiteten Substanz ein detektierbares Signal bildet, dadurch gekennzeichnet, daß Probenauftrags- und Detektionszone auf einem stapelartigen Verbund aus einem Vlies und einer porösen Membran angeordnet sind, die sich in einem einen flächigen Flüssigkeitsübergang ermöglichenden direkten oder indirekten Kontakt befinden und die Membran eine Polyamid-, Polyvinylidendifluorid-, Polyethersulfon- oder Polysulfonmembran ist, welche Flüssigkeit horizontal, das heißt in der Fläche deutlich langsamer transportiert als das Vlies.

Gegenstand der Erfindung ist auch ein stapelartiger Materialverbund aus einem Vlies und einer porösen Membran, dadurch gekennzeichnet, daß die Membran eine Polyamid-, Polyvinylidendifluorid-, Polyethersulfon- oder Polysulfonmembran ist, welche Flüssigkeit deutlich langsamer horizontal, das heißt in der Fläche transportiert als das Vlies.

Weiterhin ist Gegenstand der Erfindung die Verwendung eines wie vorstehend beschriebenen stapelartigen Materialverbundes zur Herstellung eines obigen erfindungsgemäßen Analyseelementes.

Schließlich ist Gegenstand der Erfindung ein Verfahren zur Bestimmung eines Analyten in einer Flüssigkeit mittels eine erfindungsgemäßen Analyseelementes, dadurch gekennzeichnet, daß die zu untersuchende Flüssigkeit in der Probenaufgabezone mit dem Vlies kontaktiert wird und die Bestimmung aufgrund einer Signalbildung in der Detektionszone auf der Membranseite erfolgt.

In dem erfindungsgemäßen mehrschichtigen Analysenelement sind die Probenaufgabe- und die Detektionszone nebeneinander angeordnet, das heißt, sie befinden sich dort seitlich versetzt, nicht in einer Achse angeordnet. Der Begriff der Zonen erstreckt sich auf das Analysenelement insgesamt und kann - vertikal gesehen - durch mehrere Schichten hindurchgehen. Erfindungsgemäß enthält das mehrschichtige Analysenelement der vorliegenden Erfindung einen stapelartigen Verbund aus einem Vlies und einer porösen Membran. Probenaufgabezone und Detektionszone erstrecken sich deshalb - vertikal gesehen - sowohl durch das Vlies als auch durch die Membran.

In der Probenaufgabezone wird die zu untersuchende flüssige Probe mit dem mehrschichtigen Analysenelement in Kontakt gebracht. In der Detektionszone des mehrschichtigen Analysenelementes wird ein Signal gemessen, wenn sich ein Analyt in der zu untersuchenden Flüssigkeit befindet.

Gemäß der vorliegenden Erfindung müssen sich Vlies und poröse Membran in einem einen flächigen Flüssigkeitsübergang ermöglichenden direkten oder indirekten Kontakt befinden. Ein direkter Kontakt kann dadurch erreicht werden, daß die poröse Membran auf dem Vlies oder umgekehrt das Vlies auf der porösen Membran hergestellt wird. Um. einen ausreichenden Kontakt zwischen Vlies und Membran zu gewährleisten können auch zuvor getrennt hergestellte Materialien durch Klammern, Vernähen oder Verklebung der Kanten, zusammengehalten werden. Es ist jedoch auch möglich, daß zuvor hergestelltes Vlies und Membran durch ein weiteres Material in einen dauerhaften indirekten Kontakt miteinander gebracht werden. Ein solches Material kann ein Kleber, wie beispielsweise Schmelzkleber sein, der faden- oder punktmäßig so aufgebracht wird, daß er Flüssigkeit weitgehend ungehindert zwischen Vlies- Membranschicht übertreten läßt. Solche klebenden Schichten sind beispielsweise aus EP-A-0 166 365 bekannt. Es können auch thermoplastische Gewebe oder Vliese so zwischen Vlies und Membran gelegt sein, daß nach thermischer Behandlung Vlies und Membran so laminiert sind, daß ein flächiger Flüssigkeitsübergang zwischen Vlies und Membran möglich ist. Ein solches Verfahren ist beispielsweise aus WO-A-9217768 bekannt. Dort sind auch mögliche thermoplastische Materialien aufgelistet. Erfindungsgemäß hat es sich als bevorzugt erwiesen, Vlies und poröse Membran mittels eines dünnen Netzes aus thermoplastischem Polyamid-Copolymer zu laminieren. Vorteilhafterweise handelt es sich bei dem thermoplastischen Polyamid-Copolymer um solches, das bei Temperaturen größer 100°C, ganz besonders bevorzugt zwischen 100 und 120°C schmilzt bzw. weich wird, wie beispielsweise xiro® -Web der Firma Sarnatech-Xiro AG (Schmitten, Schweiz).

Eine weitere Möglichkeit einen flächigen Verbund zwischen Vlies und Membran herzustellen, ist das Aufsprühen adhäsiver Polymere aus Schmelze oder Lösungsmittel derart, daß ein Großteil der Kontaktfläche offen bleibt.-Vor dem Aushärten muß das zweite Material, gegebenenfalls unter Druck aufgelegt werden. Hierbei entsteht eine unregelmäßige Klebestruktur auf der Kontaktfläche.

Die Art und Weise, wie und mit welchem Material das Vlies und die poröse Membran des erfindungsgemäßen stapelartigen Verbundes laminiert werden ist nur von untergeordneter Bedeutung, so lange sichergestellt ist, daß das die beiden Schichten zusammenhaltende Material chemisch inert ist und zu einem solchen Kontakt von Vlies und poröser Membran führt, daß ein homogener flächiger Flüssigkeitsübergang vom Vlies in die poröse Membran gewährleistet wird. Wesentlich wichtiger sind die Eigenschaften des Vlieses und der porösen Membran des stapelartigen Materialverbundes.

Das Vlies muß Flüssigkeit sehr schnell in der Fläche transportieren können. Als geeignet haben sich Zellulose- oder Glasfaservliese erwiesen. Glasfaservliese sind vor allem dann besonders gut geeignet, wenn die zu untersuchende Probe Vollblut ist. Glasfaservliese, wie sie beispielsweise in der europäischen Patentanmeldung 0 045 476 beschrieben sind, sind hervorragend geeignet. Dies sind vor allem Glasfaservliese aus Glasfasern mit einem mittleren Durchmesser von 0,2 bis 2,5 µm und einer Dichte von 0,1 bis 0,5 g / cm³. In solchen Glasfaservliesen wandern die Erythrozyten einer Blutprobe langsamer als das Plasma, was letztendlich zu einer Trennung von Erythrozyten und Plasma führt. Um die Abtrennung von Plasma im Vliesmaterial noch zu verbessern, kann es Erythrozyten aggregierende Substanzen tragen. Solche Substanzen sind dem Fachmann bekannt. So können hierfür beispielsweise Lektine, Antikörper gegen Erythrozyten, Aminosäuren oder auch Farbstoffe und aliphatische Diamine, wie sie beispielsweise aus der europäischen Patentanmeldung 0 133 895 bekannt sind oder kationische Polymere eingesetzt werden.

Als Membran kann erfindungsgemäße ein poröses Material eingesetzt werden, das Flüssigkeit in der Fläche deutlich langsamer transportiert als das Vlies. Dies bedeutet, daß bei einem flächigen direkten oder indirekten Kontakt von Vlies und Membran Flüssigkeit, die auf das Vlies aufgegeben wird, sich dort sehr schnell ausbreitet, bevor sie vertikal, gleichmäßig über die Kontaktfläche in die Membran aufsteigt und diese füllt. Als Membran im vorliegenden Sinne werden dünne, zusammenhängende, jedoch poröse Schichten verstanden. Erfindungsgemäß sind Polyamid-, Polyvinylidendifluorid-, Polyethersulfon- oder Polysulfonmembranen besonders bevorzugt. Ganz besonders bevorzugt sind Polyamidmembranen, insbesondere solche aus Polyamid 66. In solchen erfindungsgemäß einsetzbaren Membranen erfolgt eine Benetzung in der Fläche relativ langsam, so daß die Transportgeschwindigkeit von Flüssigkeit in die Fläche der Membran ebenfalls relativ langsam ist. Erfindungsgemäß einsetzbare Membranen können auch Stützvliese oder -gewebe aus anderen Materialien im Inneren haben, um die mechanischen Eigenschaften, wie Reißfestigkeit oder Naßausdehnung zu verbessern.

Polysulfon-, Polyethersulfon- und Polyvinylidendifluoridmembranen sind im wesentlichen dann vorteilhaft einsetzbar, wenn sie benutzungsaktiviert sind, da sie von Natur aus hydrophob sind. Diese Benetzungsaktivierung kann beispielsweise durch Legierung mit einem wasserlöslichen oder wasserquellbaren Polymer beim Membranherstellungsprozeß erfolgen. Beispielsweise kann hierzu Polyvinylpyrolidon eingesetzt werden.

Die erfindungsgemäß eingesetzte Membran soll Poren einer Porenweite zwischen 0,01 und 5µm, vorzugsweise zwischen 0,04 und 3µm aufweisen. Als bevorzugt haben sich solche Membranen erwiesen, die aus zwei Schichten aufgebaut sind, die jedoch so eng miteinander verbunden sind, daß sie nicht voneinander getrennt werden können. Die beiden Schichten unterscheiden sich in ihrer Porenweite. Die eine Schicht ist klein- die andere größerporig. In der kleinporigen Schicht der Membran beträgt die Porenweite 0,01 bis 1µm, vorzugsweise 0,04 bis 0,5 µm. Im großporigen Bereich beträgt die Porenweite 0,1 bis 5 µm, vorzugsweise 0,3 bis 3 µm. Die Porenweite im großporigen Bereich ist mindestens um den Faktor 3, bevorzugt um den Faktor 5-10 größer als die im kleinporigen Bereich der Membran. Im erfindungsgemäßen mehrschichtigen Analysenelement ist eine solche Membran, die auf gegenüberliegenden Oberflächen Poren unterschiedlicher Porenweiten besitzt so angeordnet, daß die Membranoberfläche mit den größeren Poren dem Vlies zugewandt ist.

Der Begriff "Porenweite" gibt an, daß Teilchen ab einer Größe, die der genannten Porenweite entspricht, bei einem Filtrierprozess nicht durch die Schicht hindurchdringen.

Um die Benetzbarkeit der Membran durch die aus dem Vlies in die Membran gelangende Flüssigkeit zu erhöhen, kann die Membran mit Benetzungsmitteln behandelt sein. Es kommen hierzu Detergentien oder auch wasserunlösliche Proteine in Frage. Besonders mit Benetzungsvermittler behandelte Polyvinylidendifluorid-, Polyethersulfon- und Polysulfonmembranen sind erfindungsgemäß vorteilhaft einsetzbar.

In der gesamten Probenauftragszone, die sich bis zur Detektionszone des mehrschichtigen Analysenelementes erstreckt, ist die Membran so behandelt, daß sie keine Flüssigkeit aufnimmt und transportiert. Dies kann beispielsweise dadurch erreicht werden, daß in dieser Zone Polymere so aufgebracht werden, daß die Membranporen verschlossen werden und so ein Flüssigkeitsdurchtritt aus dem Vlies in die Membran verhindert wird. Grundsätzlich sind hierfür alle Polymere geeignet, die nach dem Aufbringen auf die Membran wasserunlöslich sind. Zur Verhinderung der Flüssigkeitsaufnahme in die Membran in der Probenaufgabezone können insbesondere hydrophobe Polymere eingesetzt werden. Ein Beispiel für ein solches mögliches Polymer ist ein Copolymer aus Vinylacetat und Vinyllaurat.

Ganz besonders bevorzugt ist das erfindungsgemäße mehrschichtige Analysenelement in der Probenaufgabezone so mit Flüssigkeitsaufnahme verhinderndem Material behandelt, daß nicht nur die Membran in der Probenaufgabezone dieses Material enthält, sondern auch die Kontaktzone zwischen Membran und Vlies. Hierdurch soll verhindert werden, daß Flüssigkeit innerhalb der Probenaufgabezone des mehrschichtigen Analysenelementes sich in der Kontaktzone zwischen Membran und Vlies bewegen kann. Dies könnte beispielsweise einen störenden Einfluß auf den Einsatz des erfindungsgemäßen mehrschichtigen Analysenelementes für die Untersuchung von Blut als Probenflüssigkeit haben. Probenflüssigkeit darf deshalb in der Probenaufgabezone des erfindungsgemäßen Analysenelementes nicht mit der Membran in Kontakt kommen. Um dies zu erreichen ist es ausreichend, wenn nur der obere Teil des Vlieses, der der Membran am nächsten ist, in der Probenaufgabezone mit Flüssigkeitsaufnahme verhindernden Material behandelt ist. Aus praktischen Gesichtspunkten hat es sich jedoch als vorteilhaft erwiesen, wenn die Membran in der Probenaufgabezone in ihrem gesamten Querschnitt, die gesamte Kontaktzone zwischen Vlies und Membran in der Probenaufgabezone sowie der der Membran nächstliegende Bereich des Vlieses in der Probenaufgabezone mit Flüssigkeitsaufnahme verhinderndem Material behandelt ist.

In dem erfindungsgemäßen mehrschichtigen Analysenelement muß die Membran auch so beschaffen sein, daß sie das zur Analytbestimmung erforderliche Reagenz tragen kann. Das heißt, sie muß gegenüber diesem Reagenz inert sein, sollte es jedoch adsorbieren. Eine kovalente Bindung des Reagenzes an die Membran ist nicht erforderlich, jedoch auch nicht ausgeschlossen. Aufgrund des über die gesamte Fläche der Detektionszone gleichmäßigen Flüssigkeitsübertritts aus dem Vlies in die Membran ist es jedoch ausreichend, wenn das für die Analytbestimmung erforderliche Reagenz dort adsorbiert so vorliegt, daß es die für die Analytbestimmung notwendige Signalbildung mit dem Analyt eingeht.

Zur Signalbildung kommen insbesondere Farbänderung, Farbbildung oder Farbabnahme in Frage. Es können jedoch auch solche Reagenzien eingesetzt werden, die bei Anwesenheit des Analyten Fluoreszenz erzeugen oder abnehmen lassen. Farbbildung oder Farbveränderung sind jedoch die bevorzugten Signalbildungsmethoden. Je nach Analyt sind dem Fachmann hierzu geeignete Reagenzien bekannt.

Das Reagenz befindet sich auf dem in die Detektionszone des erfindungsgemäßen mehrschichtigen Analysenelementes hineinragenden Teil der Membran. Zwar kann das Reagenz grundsätzlich in dem gesamten Querschnitt der Membran enthalten sein, vorteilhafterweise ist das Reagenz jedoch so auf bzw. in die Membran gebracht, daß sich das Reagenz auf der dem Vlies abgewandten Seite der Membran befindet. Idealerweise ist die dem Vlies nächstliegende Oberfläche der Membran reagenzfrei.

Während es für die Bestimmung eines Analyts möglich ist, daß sich in der gesamten äußeren Oberfläche der Membran, die sich in der Detektionszone befindet, Reagenz enthalten ist, ist es auch möglich, dieses Reagenz nur auf einen Teil der Detektionszone in der Membran zu beschränken. So ist es auch möglich, zur Bestimmung mehrerer Analyte in einer flüssigen Probe mehrere unterschiedliche Reagenzien nebeneinander in der Detektionszone der Membran unterzubringen. In einem solchen Fall können die für unterschiedliche Analyte spezifischen Testfelder innerhalb der Detektionszone räumlich voneinander getrennt vorliegen, das heißt, sie berühren sich nicht, sondern sind durch reagenzfreie Bereiche voneinander getrennt oder sie können so dicht benachbart sein, daß sie sich berühren. Insbesondere bei einem solchen erfindungsgemäß mehrschichtigen Analyseelement, das zur Bestimmung mehrere Analyte in einer Flüssigkeit geeignet ist, hat es sich als vorteilhaft erwiesen, daß durch die erfindungsgemäße Anordnung eine horizontale Chromatographie der Flüssigkeit in der Membran, das heißt, eine Wanderung der Flüssigkeit in der Membranfläche weitgehend vermieden wird. Dadurch, daß sich Flüssigkeit schnell im Vlies ausbreitet und dann anschließend Flüssigkeit homogen über die Kontaktzone zwischen Vlies und Membran flächig in die Membran übertritt, gibt es keine Vermischung der Reagenzien in den nebeneinander angeordneten Testbezirken für verschiedenen Analyte.

Unter Umständen sind zur Bestimmung eines Analyten mehrere Reaktionsschritte erforderlich und das hierfür erforderliche Reagens beinhaltet Substanzen, die miteinander nicht verträglich sind, oder aus anderen Gründen sollen Reagenzbestandteile räumlich getrennt angeordnet sein. In einem solchen Fall ist es möglich, in der Membran eventuell nur die Reagenzbestandteile unterzubringen, die mit einer von dem zu bestimmenden Analyt abgeleiteten Substanz ein detektierbares Signal bilden. Die übrigen Reagenzbestandteile können dann beispielsweise auch so im Vlies enthalten oder aufgebracht sein, daß Analyt dort zunächst in eine vom Analyt abgeleitete Substanz überführt wird und diese dann erst in der Membran zur detektierbaren Signalbildung führt. So ist es vorstellbar, daß beispielsweise Triglyceride in einer im Vlies ablaufenden Vorreaktion so gespalten werden, daß Glycerin als von Triglycerid abgeleitete Substanz in der Membran zu einem detektierbaren Signal führt, das mit der Menge an Triglycerid in der Probe korreliert.

Ebenso können im Vlies auch Entstörreaktionen, wie beispielsweise die Beseitigung von Ascorbinsäure in der Probenflüssigkeit durch im Vlies enthaltene Reagenzbestandteile, beispielsweise Jodat oder Ascorbatoxidase, durchgeführt werden. Auch Haltbarkeitsgründe können ausschlaggebend dafür sein, daß Reagenzbestandteile räumlich getrennt in Vlies und Membran aufgeteilt eingesetzt werden. Die im Vlies untergebrachten Reagenzbestandteile können dann in der Probenflüssigkeit angereichert und in die Detektionszone mittransportiert werden, wo dann alle zum Analytnachweis nötigen Reagenzbestandteile vorliegen, so daß dort die Nachweisreaktion gestartet wird und abläuft.

Aufgrund der vorteilhaften Eigenschaften, die der vorstehend beschriebene stapelartige Materialverbund aus Vlies und poröser Membran bietet, soll gerade dieser stapelartige Materialverbund ebenfalls Gegenstand der Erfindung sein.

Wie ebenfalls bereits vorstehend beschrieben, kann der stapelartige Materialverbund zur Herstellung eines erfindungsgemäßen mehrschichtigen Analysenelementes verwendet werden. Hierzu kann der stapelartige Materialverbund gegebenenfalls noch besser handhabbar gemacht werden, in dem er beispielsweise auf eine steife Trägerfolie aufgebracht wird, so daß der stapelartige Materialverbund leicht und hygienisch mit der zu untersuchenden Probe in Kontakt gebracht werden kann. Die Trägerfolie kann der Membran benachbart sein, so daß Probenflüssigkeit auf die frei zugängliche Probenaufgabezone des Vlieses aufgebracht werden kann. Um die Detektionszone in der Membran vermessen zu können ist es notwendig, daß in einem solchen Fall die Trägerfolie in der Detektionszone perforiert oder transparent ist. Im Falle von Löchern sollen diese natürlich an der Stelle sein, in der sich der bzw. die Reagenz enthaltenden Bereiche oder Testfelder befinden. Die Größe der Löcher muß dementsprechend der Testfeldgröße angepaßt sein.

Ein erfindungsgemäßes mehrschichtiges Analysenelement ist jedoch auch denkbar, bei dem sich der stapelartige Materialverbund aus Vlies und poröser Membran in einem festen Gehäuse, beispielsweise aus Kunststoffbefindet. Dieses Gehäuse muß zumindest eine Öffnung besitzen, durch die Probenflüssigkeit mit der Probenaufgabezone kontaktiert werden kann. Analog wie vorstehend für die Trägerfolie beschrieben, kann auch im Falle des Gehäuses der der Membran benachbarte Teil zumindest im Bereich der Detektionszone transparent oder perforiert sein.

In einem erfindungsgemäßen mehrschichtigen Analysenelement kann die Probe direkt auf die Probenaufgabezone aufgebracht werden. Es können jedoch auch Ausführungsformen vorliegen, bei denen die Flüssigkeit zunächst nicht direkt mit der Probenaufgabezone kontaktiert wird sondern zu dieser erst nach Durchlaufen einer bestimmten Strecke, beispielsweise eines kapillaren Kanals, gelangt.

Nachdem Flüssigkeit das Vlies des erfindungsgemäßen stapelartigen Materialverbundes in der Probenaufgabezone kontaktiert hat, verteilt sich die Flüssigkeit schnell über die gesamte der Flüssigkeit zugängliche Fläche des Vlieses. Wenn das Vlies Flüssigkeit enthält, dringt Flüssigkeit gleichmäßig, das heißt, homogen über die gesamte Fläche in die darüberliegende Membran. Da in der Membranfläche Flüssigkeit deutlich langsamer transportiert wird als in der Vliesfläche ist das Auffüllen der Membranfläche mit Flüssigkeit vor allem durch den zur Ausbreitung in der Fläche quergerichteten Flüssigkeitsübergang vom Vlies in die Membran bedingt. Wenn dieser Flüssigkeitsübergang von Vlies in Membran durch eine wasserundurchlässige Schicht in der Probenaufgabezone nicht möglich ist, erfolgt der Flüssigkeitsübertritt nur in der Detektionszone. Dort kommt die Probenflüssigkeit mit dem oder den Reagenzien in dem oder den Testfeldern der Membran in Berührung, worauf sich bei Anwesenheit des oder der zu bestimmenden Analyte in den Testfeldern ein Signal bildet, das visuell oder apparativ beobachtet wird. Im Falle der Verwendung von Blut als Probenflüssigkeit und einer entsprechenden Auswahl des Vliesmaterials, wie beispielsweise Glasfaser bzw. eines entsprechenden Einsatzes von Erythrozyten aggregierenden Substanzen in der Probenauftragszone des Vlieses findet im Vlies eine Trennung von Erythrozyten und Plasma statt, so daß lediglich Plasma in die in der Membran befindlichen Testfelder in der Detektionszone des mehrschichtigen Analysenelementes gelangt.

In Figur 1 ist eine Ausführungsform eines stapelartigen Materialverbundes in einem erfindungsgemäßen Analysenelement dargestellt.

Figur 2 stellt eine mögliche Ausführungsform eines erfindungsgemäßen mehrschichtigen Analysenelementes dar.

Figur 3 gibt eine weitere mögliche Ausführungsform eines erfindungsgemäßen mehrschichtigen Analysenelementes wieder.

Figur 4 zeigt eine alternative Ausführungsform eine erfindungsgemäßen mehrschichtigen Analyseelementes.

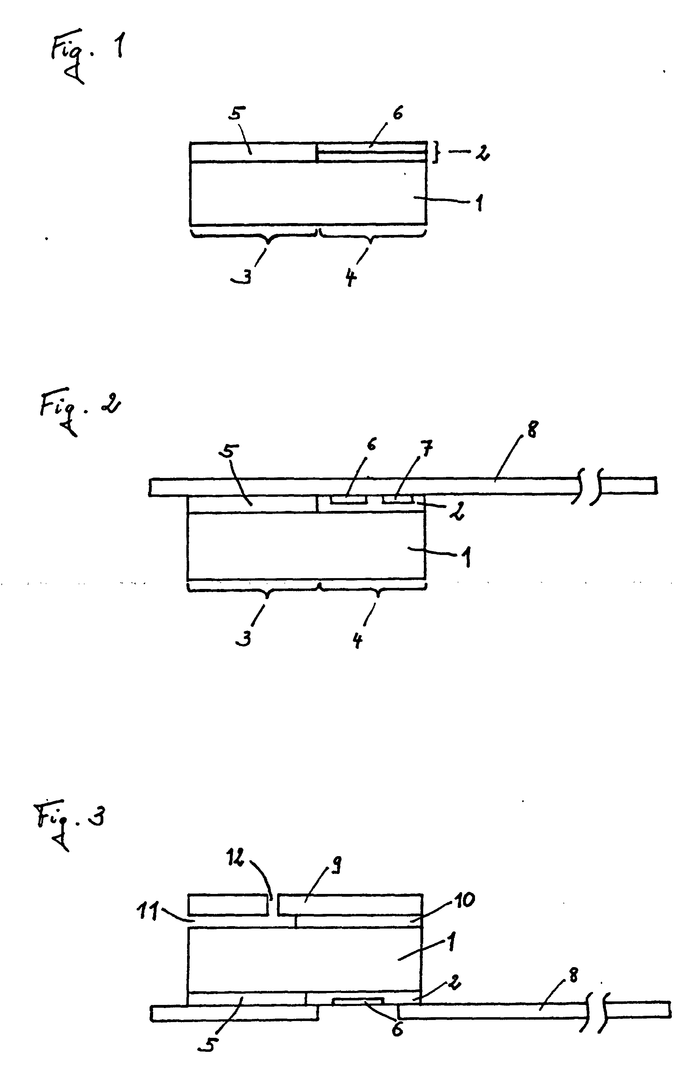

In Figur 1 ist ein bevorzugter erfindungsgemäßer stapelartiger Materialverbund aus Vlies (1) und Membran (2) dargestellt. Die Probenaufgabezone (3) erstreckt sich über den Bereich, der durch den flüssigkeitsundurchlässigen Bereich (5) der Membran (2) definiert ist. Flüssigkeit, die in der Probenaufgabezone (3) auf das Vlies aufgebracht wird, wird so auf jeden Fall durch den flüssigkeitsundurchlässigen Bereich (5) daran gehindert, dort in die Membran (2) einzudringen. Ein Übertritt von Flüssigkeit aus dem Vlies (1) in die Membran (2) ist nur innerhalb der Detekionszone (4) möglich. Durch die zuvor geschilderte Materialauswahl verteilt sich auf das Vlies (1) in der Probenaufgabezone (3) aufgegebene Flüssigkeit schnell innerhalb des Vlieses (1) und gelangt von dort quer zur Ausbreitungsrichtung in der Vliesfläche in die Detektionszone (4) der Membran (2) und tritt dort in den Reaganz enthaltenden Bereich (6) ein. Bei Anwesenheit des Analyts findet dort eine Signalbildung statt, die von der Membranseite her visuell oder apparativ beobachtet werden kann.

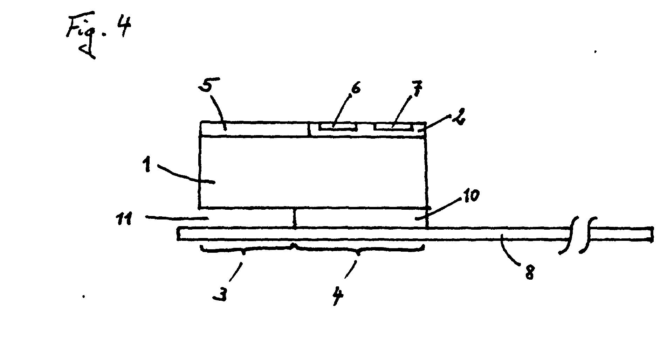

In Figur 2 ist eine Ausführungsform eines erfindungsgemäßen mehrschichtigen Analysenelementes dargestellt, bei dem der stapelartige Materialverbund aus Vlies (1) und Membran (2) auf einer steifen Trägerfolie (8) befestigt ist. Als steife Trägerfolien kommen alle möglichen inerten steifen Materialien in Frage, die die Handhabbarkeit des Materialverbundes erleichtern. Mögliche Materialien können Glas, Karton oder Kunststoff sein. Steife Kunststoffolien sind besonders bevorzugt.

In dem in Figur 2 dargestellten erfindungsgemäßen mehrschichtigen Analysenelement sind in der Membran (2) zwei Reagenz enthaltende Bereiche (6, 7) dargestellt, die es ermöglichen, zwei unterschiedliche Analyte in der zu untersuchenden Probenflüssigkeit zu detektieren. Im dargestellten Fall ist die Trägerfolie (8) in der Detektionszone nicht perforiert und muß deshalb dort transparent sein, um eine Signalbildung in der Detektionszone (4) der Membran (2) im Bereich der Testfelder (6,7) beobachten zu können.

In Figur 3 ist eine weitere mögliche Ausführungsform eines erfindungsgemäßen mehrschichtigen Analysenelementes dargestellt. Hier befindet sich der stapelartige Materialverbund aus Vlies (1) und Membran (2) auf einer Trägerfolie (8), die im Bereich des Reagenz enthaltenden Testfeldes (6) ein Loch enthält. Auf der Vliesseite ist eine Deckfolie (9) so mit einem Abstandshalter (10) angebracht, daß sich in der Probenaufgabezone ein Kapillarspalt (11) befindet.

Als Deckfolie (9) können inerte ausreichend steife Materialien zum Einsatz kommen, wie beispielsweise Kunststoffolien. Auch bei dem Material für den Abstandshalter (10) kommt es darauf an, daß es gegenüber der Probenflüssigkeit und dem Analyt inert ist. Außerdem sollen sowohl die Deckfolie (9) als auch der Abstandshalter (10) keine Flüssigkeit oder gar den Analyt aufnehmen. Insofern sollten hierfür geeignete Materialien nicht absorbierend sein.

Wird der Kapillarspalt (11) mit Probenflüssigkeit kontaktiert, wird Flüssigkeit schnell den gesamten Kapillarspalt (11) füllen. Luft kann hierbei aus der Luftaustrittsöffnung (12) austreten. Vom Kapillarspalt (11) tritt Flüssigkeit in die Probenaufgabezone des Vlieses (1) über und verteilt sich dort rasch. Wegen des flüssigkeitsundurchlässigen Bereiches (5) in der Membran (2) und der der Membran nächstliegenden Vliesoberfläche kann Flüssigkeit nur im Bereich der Detektionszone aus dem Vlies (1) in die Membran (2) übertreten und dort im Testfeld (6) bei Anwesenheit des zu bestimmenden Analyten in der Probenflüssigkeit ein Signal bilden, das visuell oder apparativ beobachtet werden kann. Bei einem seitlich offenen Kapillarspalt (11) ist eine Luftaustrittsöffnung (12) nicht unbedingt erforderlich. Nur bei einem Kanal (11) der nur an der Einlaßseite offen ist, ist eine Entlüftungsöffnung (12) notwendig.

Figur 4 zeigt eine alternative bevorzugte Ausführungsform eines erfindungsgemäßen mehrschichtigen Analysenelementes. Hier ist der stapelartige Materialverbund aus Vlies (1) und Membran (2) über einen Abstandshalter (10) auf der Trägerfolie (8) befestigt. Messungen erfolgen auf der freizugänglichen Membran (2).

Mit dem beschriebenen erfiindungsgemäßen stapelartigen Materialverbund bzw. dem erfindungsgemäßen mehrschichtigen Analysenelement ist es möglich, in einer Reaktionsschicht eine flächenhomogene Reaktion ohne chemische Fixierung der Reagenzien durchzuführen. Die Kombination eines schnell Flüssigkeit transportierenden Vlieses und einer aus dem Vlies über die gesamte Fläche homogen Flüssigkeit aufnehmenden porösen Membran vermeidet die im Stand der Technik auftretenden Chromatographieeffekte durch eine Anreicherung von Reagenzien in einer Flüssigkeitsfront. Ebenso wird auch der Effekt vermieden, daß Analyt in einer Flüssigkeitsfront schneller und damit stärker abreagiert als in der dahinter folgenden Flüssigkeit.

Das erfindungsgemäße mehrschichtige Analysenelement ist kompakt, einfach und billig herzustellen. In einem schrittweisen Herstellungsverfahren ist es möglich, zuerst den stapelartigen Materialverbund herzustellen bevor teuere Reagenzien aufgebracht werden. So ist das Kostenrisiko, daß bei der Montage des Materialverbundes ein Fehler auftritt, gegenüber dem bisher üblichen Verfahren, daß jede Schicht gebrauchsfertig, das heißt, gegebenenfalls teueres Reagenz enthaltend montiert wird, minimiert. Dadurch, daß der stapelartige Materialverbund ohne Reagenz als Halbfertigerzeugnis so verwendet werden kann, daß er erst nach Fertigstellung mit den zur jeweiligen Analytbestimmung erforderlichen Reagenzien behandelt wird, ist dieser reagenzfreie Materialverbund universal nutzbar.

Die Herstellung eines erfindungsgemäßen mehrschichtigen Analysenelementes kann so erfolgen, daß zunächst ein Verbund aus Vlies und Membran hergestellt wird. Der Verbund kann dadurch erfolgen, daß Vlies und Membran durch Klammern, Kantenverklebung, Vernähen oder durch eine dazwischenliegende thermoplastische Schicht miteinander laminiert werden. Anschließend kann die Membran des Materialverbundes im Bereich der Probenauftragszone so behandelt werden, daß die Membran und die der Membran benachbarte Schicht des Vlieses für die Probenflüssigkeit undurchlässig wird. Dies kann beispielsweise durch nichtsättigungstränkende Imprägnierung dieses Membranbereiches mittels einer Kunststofflösung, -emulsion oder -suspension erreicht werden. In einem solchen Fall muß dafür Sorge getragen werden, daß das verwendete Lösungs- oder Dispergiermittel die Membran, das Vlies und den Zusammenhalt beider Materialien nicht negativ beeinflußt. Obwohl dies grundsätzlich natürlich auch schon bereits vor der Laminierung des Vlieses mit der Membran möglich ist, erfolgt eine Belegung des Vlieses mit Substanzen, wie beispielsweise Benetzungsvermittler, erythrozytenaggregierende Substanz, die Nachweisreaktion entstörenden Substanzen oder Reagenzbestandteilen, die mit den übrigen in die Membran einzubringenden Reagenzbestandteilen nicht verträglich sind in der Probenauftragszone mit nichtsättigungstränkenden Verfahren vorteilhafterweise nach der Laminierung von Vlies und Membran. Ebenso wird in die Detektionszone der Membran das bzw. die Reagenzien für die Bestimmung des bzw. der Analyte nicht sättigungstränkend am Materialverbund aufgebracht. Das Auf- bzw. Einbringen der Substanzen mit nichtsättigungstränkenden Verfahren erfolgt flächendefiniert, das heißt, nach Abschluß des Verfahrens liegen die Substanzen nur in bestimmten Bereichen von Vlies oder Membran vor, nicht jedoch in dem gesamten Material. Durch die nichtsättigungstränkenden Verfahren ist es möglich, die Eindringtiefe der Substanzen zu regulieren. So ist es möglich, Reagenzien auf das Vlies bzw. die Membran des Materialverbundes zu beschränken. Nicht sättigungstränkende Verfahren sind beispielsweise Druckverfahren, wie Siebdruck, die Düsenimprägnierung, das Sprühen, Ink-Jet oder Strichimprägnierungsverfahren mit eine Rolle. Besonders das Rollenverfahren kann für die Herstellung des erfindungsgemäßen mehrschichtigen Analysenelementes vorteilhaft eingesetzt werden. Hierbei läuft ein Teil einer Rolle durch die Flüssigkeit, die auf den Materialverbund aufgebracht werden soll. Der Materialverbund selbst wird mittels einer Trägerwalze mit der durch die Tränklösung laufenden Auf-. tragsrolle so in Kontakt gebracht, daß die Auftragsrolle durch den vorbeiziehenden Materialverbund in Bewegung gehalten wird. So wird beim Abrollen der Auftragsrolle auf den Materialverbund Flüssigkeit auf Vlies oder Membran in der Breite der Auftragsrolle aufgebracht. Anschließend wird der behandelte Materialverbund getrocknet und kann dann so zerschnitten werden, daß er in ein Kunststoffgehäuse zum Fertigen mehrschichtigen Analysenelementes eingelegt werden kann. Es ist aber auch möglich, daß vor dem Zerschneiden des wie vorstehend beschriebenen hergestellten Materialverbundes noch eine Trägerfolie auf die Membran aufgebracht wird. Ein anschließendes Schneiden des Materialverbundes quer zur Laufrichtung des Materialverbundbandes ergibt so fertige einsatzfähige mehrschichtige Analysenelemente.

## Patentansprüche

1. Mehrschichtiges Analysenelement zur Bestimmung eines Analyten in einer Flüssigkeit enthaltend nebeneinander angeordnet eine Probenauftragszone und eine Detektionszone, wobei die Detektionszone ein Reagenz enthält, das mit dem zu bestimmenden Analyt oder einer hiervon abgeleiteten Substanz ein detektierbares Signal bildet,
**dadurch gekennzeichnet, daß** Probenauftrags- und Detektionszone auf einem stapelartigen Verbund aus einem Vlies und einer porösen Membran angeordnet sind, die sich in einem einen flächigen Flüssigkeitsübergang ermöglichenden direkten oder indirekten Kontakt befinden und die Membran eine Polyamid-, Polyvinylidendifluorid-, Polyethersulfon- oder Polysulfonmembran ist, welche Flüssigkeit in der Fläche deutlich langsamer transportiert als das Vlies.

2. Mehrschichtiges Analysenelement gemäß Anspruch 1, **dadurch gekennzeichnet, daß** die Membran aus einer klein- und einer großporigen Schicht besteht, die übereinander angeordnet sind.

3. Mehrschichtiges Analysenelement gemäß Anspruch 1 oder 2, **dadurch gekennzeichnet, daß** sich Reagenz in oder auf der Membran befindet.

4. Mehrschichtiges Analysenelement gemäß einem der Ansprüche 1-3, **dadurch gekennzeichnet, daß** in der Probenauftragszone die Membran so behandelt ist, daß sie keine Flüssigkeit aufnimmt.

5. Mehrschichtiges Analysenelement gemäß Anspruch 3 oder 4, **dadurch gekennzeichnet, daß** sich Reagenz dort in oder auf der Membran befindet, wo die Membran Flüssigkeit aufnehmen kann.

6. Mehrschichtiges Analysenelement gemäß Anspruch 4 oder 5, **dadurch gekennzeichnet, daß** die Membran Reagenz zur Bestimmung unterschiedlicher Analyte räumlich getrennt voneinander enthält.

7. Mehrschichtiges Analysenelement gemäß einem der vorhergehenden Patentansprüche, **dadurch gekennzeichnet, daß** die Poren der Membran eine Porenweite zwischen 0,01 und 5 µm, vorzugsweise zwischen 0,04 und 3 µm aufweisen.

8. Stapelartiger Materialverbund aus einem Vlies und einer porösen Membran, **dadurch gekennzeichnet, daß** die Membran eine Polyamid-, Polyvinylidendifluorid-, Polyethersulfon- oder Polysulfonmembran ist, welche Flüssigkeit deutlich langsamer in der Fläche transportiert als das Vlies.

9. Verwendung eines stapelartigen Materialverbundes gemäß Patentanspruch 8 zur Herstellung eines mehrschichtigen Analysenelementes gemäß einem der Patentansprüche 1-7.

10. Verfahren zur Bestimmung eines Analyten in einer Flüssigkeit mittels eines mehrschichtigen Analysenelementes gemäß einem der Patentansprüche 1-7, **dadurch gekennzeichnet, daß** die zu untersuchenden Flüssigkeit in der Probenaufgabezone mit dem Vlies kontaktiert wird und die Bestimmung aufgrund einer Signalbildung in der Detektionszone auf der Membranseite erfolgt.

## Claims

1. Multilayer analytical element for the determination of an analyte in a liquid containing a sample application zone and a detection zone which are arranged side by side in which the detection zone contains a reagent which forms a detectable signal with the analyte to be determined or with a substance derived therefrom,
wherein the sample application zone and detection zone are arranged on a pile-like complex composed of a fleece and a porous membrane which are in a direct or indirect contact that enables passage of liquid over the area and the membrane is a polyamide, polyvinylidene difluoride, polyether sulfone or polysulfone membrane which transports liquid significantly slower over the area than the fleece.

2. Multilayer analytical element as claimed in claim 1, wherein the membrane is composed of a small-pore and a large-pore layer which are arranged on top one another.

3. Multilayer analytical element as claimed in claim 1 or 2, wherein the reagent is present in or on the membrane.

4. Multilayer analytical element as claimed in one of the claims 1-3, wherein the membrane is treated in the sample application zone in such a way that it does not take up any liquid.

5. Multilayer analytical element as claimed in claim 3 or 4, wherein reagent is located in or on the membrane at a site at which the membrane can take up liquid.

6. Multilayer analytical element as claimed in claim 4 or 5, wherein the membrane contains a reagent for the determination of different analytes which are spatially separated from one another.

7. Multilayer analytical element as claimed in one of the previous claims, wherein the pores of the membrane have a pore size between 0.01 and 5 *µ*m preferably between 0.04 and 3 *µ*m.

8. Pile-like material complex made of a fleece and a porous membrane, wherein the membrane is a polyamide, polyvinylidene difluoride, polyether sulfone or polysulfone membrane that transports liquid over the area significantly slower than the fleece.

9. Use of a pile-like material complex as claimed in claim 8 for the production of a multilayer analytical element as claimed in one of the claims 1-7.

10. Method for the determination of an analyte in a liquid by means of a multilayer analytical element as claimed in one of the claims 1-7, wherein the liquid to be examined is contacted with the fleece in the sample application zone and the determination is carried out based on the signal formation in the detection zone on the membrane side.

## Revendications

1. Elément d'analyse à plusieurs couches pour la détermination d'un analyte dans un liquide, contenant disposées l'une à côté de l'autre une zone d'application d'échantillon et une zone de détection, la zone de détection contenant un réactif qui forme un signal détectable avec l'analyte à déterminer ou avec une substance qui en est dérivée, **caractérisé en ce que** la zone d'application de l'échantillon et la zone de détection sont disposées sur un composite configuré en empilement constitué d'un feutre et d'une membrane poreuse qui se trouvent en contact direct ou indirect permettant un transfert du liquide par la surface, et la membrane est une membrane de polyamide, de difluorure de polyvinylidène, de polyéthersulphone ou de polysulphone qui transporte le liquide dans la surface de manière nettement plus lente que le feutre.

2. Elément d'analyse à plusieurs couches selon la revendication 1, **caractérisé en ce que** la membrane est constituée d'une couche à petits pores et d'une couche à gros pores qui sont disposées l'une au-dessus de l'autre.

3. Elément d'analyse à plusieurs couches selon la revendication 1 ou 2, **caractérisé en ce que** le réactif se trouve dans ou sur la membrane.

4. Elément d'analyse à plusieurs couches selon l'une des revendications 1 à 3, **caractérisé en ce que** dans la zone d'application de l'échantillon, la membrane est traitée de manière à ne recevoir aucun liquide.

5. Elément d'analyse à plusieurs couches selon la revendication 3 ou 4, **caractérisé en ce que** le réactif se trouve dans ou sur la membrane là où la membrane peut recevoir du liquide.

6. Elément d'analyse à plusieurs couches selon la revendication 4 ou 5, **caractérisé en ce que** la membrane contient, séparés spatialement l'un de l'autre, des réactifs pour la détermination de différents analytes.

7. Elément d'analyse à plusieurs couches selon l'une des revendications précédentes, **caractérisé en ce que** les pores de la membrane présentent des pores dont le diamètre est compris entre 0,01 et 5 µm, de préférence entre 0,04 et 3 µm.

8. Matériau composite configuré en empilement, constitué d'un feutre et d'une membrane poreuse, **caractérisé en ce que** la membrane est une membrane de polyamide, de difluorure de polyvinylidène, de polyéthersulphone ou de polysulphone qui transporte le liquide dans la surface de manière nettement plus lente que le feutre.

9. Utilisation d'un matériau composite configuré en empilement selon la revendication 8 pour la préparation d'un élément d'analyse à plusieurs couches selon l'une des revendications 1 à 7.

10. Procédé de détermination d'un analyte dans un liquide au moyen d'un élément d'analyse à plusieurs couches selon l'une des revendications 1 à 7, **caractérisé en ce que** le liquide à étudier est mis en contact avec le feutre dans la zone d'application de l'échantillon, et la détermination s'effectue sur base de la formation d'un signal dans la zone de détection du côté de la membrane.
